# FASCICULE DE BREVET EUROPEEN

(11) **EP 2 555 611 B1**
(45) Date de publication et mention de la délivrance du brevet: **21.12.2016**
(21) Numéro de dépôt: 11719663.4
(22) Date de dépôt: 06.04.2011
(51) Int. Cl.: A01K 67/027, C07K 14/47, C12N 15/85, A61K 38/17

(54) **PRÉPARATION DE PROTÉINE PLASMATIQUE DE TRANSFERT DES PHOSPHOLIPIDES (PLTP) HUMAINE RECOMBINANTE A PARTIR DU LAIT D'ANIMAUX TRANSGÉNIQUES**
ZUBEREITUNG VON REKOMBINANTEM MENSCHLICHEM PLASMAPHOSPHOLIPIDTRANSFERPROTEIN (PLTP) AUS DER MILCH VON TRANSGENEN TIEREN
PREPARATION OF RECOMBINANT HUMAN PLASMA PHOSPHOLIPID TRANSFER PROTEIN (PLTP) FROM THE MILK OF TRANSGENIC ANIMALS

(30) Priorité: 09.04.2010 FR 1001490
(43) Date de publication de la demande: 13.02.2013
(73) Titulaire: Bioprotein Technologies SA, 92100 Boulogne-Billancourt (FR); Institut National de la Santé et de la Recherche Médicale, 75013 Paris (FR)
(72) Inventeur: RIPOLL, Pierre-Jean, F-95140 Mennecy (FR); LAGROST, Laurent, F-21000 Dijon (FR)
(74) Mandataire: Gevers & Orès
(86) Numéro de dépôt international: PCT/IB2011/051482
(87) Numéro de publication internationale: WO 2011/125034

(56) Documents cités:
- WO-A1-95/34289
- WO-A1-97/42835
- WO-A2-2007/138199
- GUO ZIWEN ET AL: "Secretion of phospholipid transfer protein by human hepatoma cell line, Hep G2, is enhanced by sodium butyrate", JOURNAL OF NUTRITION, vol. 129, no. 11, novembre 1999 (1999-11), pages 1984-1991, XP002599361, ISSN: 0022-3166 cité dans la demande
- ECHELARD Y ET AL: "Production of recombinant therapeutic proteins in the milk of transgenic animals", BIOPHARM INTERNATIONAL, ADVANSTAR COMMUNICATIONS, DULUTH, MN, US, vol. 19, no. 8, 1 août 2006 (2006-08-01), pages 36-46, XP009112476, ISSN: 1542-166X
- HOUDEBINE ET AL: "Production of pharmaceutical proteins by transgenic animals", COMPARATIVE IMMUNOLOGY, MICROBIOLOGY AND INFECTIOUS DISEASES, PERGAMON PRESS, OXFORD, GB LNKD- DOI:10.1016/J.CIMID.2007.11.005, vol. 32, no. 2, 1 mars 2009 (2009-03-01), pages 107-121, XP025926097, ISSN: 0147-9571 [extrait le 2008-02-19] cité dans la demande
- JIANGLIN FAN ET AL: "Transgenic rabbits as therapeutic protein bioreactors and human disease models", PHARMACOLOGY AND THERAPEUTICS, ELSEVIER, GB LNKD- DOI:10.1016/S0163-7258(03)00069-X, vol. 99, no. 3, 1 septembre 2003 (2003-09-01), pages 261-282, XP008091395, ISSN: 0163-7258
- HAPEREN VAN R ET AL: "HUMAN PLASMA PHOSPHOLIPID TRANSFER PROTEIN INCREASES THE ANTIATHEROGENIC POTENTIAL OF HIGH DENSITY LIPOPROTEINS IN TRANSGENIC MICE", ARTERIOSCLEROSIS, THROMBOSIS, AND VASCULAR BIOLOGY, LIPPINCOTT WILLIAMS & WILKINS, US, vol. 20, no. 4, 1 avril 2000 (2000-04-01), pages 1082-1088, XP000915014, ISSN: 1079-5642
- KEEFER C L: "Production of bioproducts through the use of transgenic animal models", ANIMAL REPRODUCTION SCIENCE, vol. 82-8, no. July, juillet 2004 (2004-07), pages 5-12, XP002599362, ISSN: 0378-4320
- DIMOND P F: "Transgenic Technology in the Production of therapeutic proteins", INNOVATIONS IN PHARMACEUTICAL TECHNOLOGY, SAMEDAN LTD, GB, [Online] 1 janvier 2000 (2000-01-01), pages 92-97, XP001525332, ISSN: 1471-7204
- K. KOLES: "N- and O-glycans of recombinant human C1 inhibitor expressed in the milk of transgenic rabbits", GLYCOBIOLOGY, vol. 14, no. 1, 26 September 2003 (2003-09-26), pages 51-64, XP055099168, DOI: 10.1093/glycob/cwh010
- GIL GEUN-CHEOL ET AL: "Analysis of the N-glycans of recombinant human Factor IX purified from transgenic pig milk", GLYCOBIOLOGY, OXFORD UNIVERSITY PRESS, US, vol. 18, no. 7, 1 July 2008 (2008-07-01), pages 526-539, XP002515191, ISSN: 0959-6658, DOI: 10.1093/GLYCOB/CWN035 [retrieved on 2008-05-02]
- EDMUNDS T ET AL: "TRANSGENICALLY PRODUCED HUMAN ANTITHROMBIN: STRUCTURAL AND FUNCTIONAL COMPARISON TO HUMAN PLASMA-DERIVED ANTITHROMBIN", BLOOD, AMERICAN SOCIETY OF HEMATOLOGY, US, vol. 91, no. 12, 15 June 1998 (1998-06-15) , pages 4561-4571, XP000892719, ISSN: 0006-4971
- K. KOLES: "Influence of lactation parameters on the N-glycosylation of recombinant human C1 inhibitor isolated from the milk of transgenic rabbits", GLYCOBIOLOGY, vol. 14, no. 11, 30 June 2004 (2004-06-30) , pages 979-986, XP055163482, ISSN: 0959-6658, DOI: 10.1093/glycob/cwh127
- RAJU T S ET AL: "Species-specific variation in glycosylation of IgG: evidence for species-specific sialylation and branch-specific galactosylation and importance for engineering recombinant glycoprotein therapeutics", GLYCOBIOLOGY, OXFORD UNIVERSITY PRESS, US, vol. 10, no. 5, 1 January 2000 (2000-01-01), pages 477-486, XP002964086, ISSN: 0959-6658, DOI: 10.1093/GLYCOB/10.5.477
- A R TALL ET AL: "Separation of a plasma phospholipid transfer protein from cholesterol ester/phospholipid exchange protein", JOURNAL OF BIOLOGICAL CHEMISTRY, vol. 258, no. 4, 25 February 1983 (1983-02-25), pages 2174-2180, XP055237969, US ISSN: 0021-9258
- L LAGROST ET AL: "Comparative study of phospholipid transfer activities mediated by cholesteryl ester transfer protein and phospholipid transfer protein", JOURNAL OF LIPID RESEARCH, vol. 35, no. 5, 1 May 1994 (1994-05-01), page 825, XP055237971, US ISSN: 0022-2275
- JOHN J ALBERS ET AL: "Impact of site-specific N-glycosylation on cellular secretion, activity and specific activity of the plasma phospholipid transfer protein", BIOCHIMICA ET BIOPHYSICA ACTA (BBA) - PROTEINS & PROTEOMICS, ELSEVIER, NETHERLANDS, vol. 1814, no. 7, 11 April 2011 (2011-04-11), pages 908-911, XP028218724, ISSN: 1570-9639, DOI: 10.1016/J.BBAPAP.2011.04.004 [retrieved on 2011-04-16]
- VALÉRIE GUYARD-DANGREMONT ET AL: "Phospholipid and cholesteryl ester transfer activities in plasma from 14 vertebrate species. Relation to atherogenesis susceptibility", COMPARATIVE BIOCHEMISTRY AND PHYSIOLOGY. PART B, BIOCHEMISTRYAND MOLECULAR BIOLOGY, vol. 120, no. 3, 1 July 1998 (1998-07-01), pages 517-525, XP055237975, GB ISSN: 1096-4959, DOI: 10.1016/S0305-0491(98)10038-X
- TOMOICHIRO OKA ET AL: "Distribution of phospholipid transfer protein in human plasma: presence of two forms of phospholipid transfer protein, one catalytically active and the other inactive", JOURNAL OF LIPID RESEARCH, vol. 41, no. 10, 1 October 2000 (2000-10-01), page 1651, XP055237978, US ISSN: 0022-2275
- M. KARKKAINEN: "Isolation and Partial Characterization of the Inactive and Active Forms of Human Plasma Phospholipid Transfer Protein (PLTP)", JOURNAL OF BIOLOGICAL CHEMISTRY, vol. 277, no. 18, 26 April 2002 (2002-04-26), pages 15413-15418, XP055237980, US ISSN: 0021-9258, DOI: 10.1074/jbc.M112247200

## Description

La présente invention est relative à la préparation de protéine plasmatique de transfert des phospholipides (PLTP) humaine recombinante à partir du lait d'animaux transgéniques, et ses utilisations.

La protéine plasmatique de transfert des phospholipides (*phospholipid transfer protein*, PLTP) a été initialement identifiée pour sa capacité à échanger des phospholipides entre les lipoprotéines circulantes et à modifier la structure et la composition des lipoprotéines pro- ou antiathérogènes. Elle appartient à la famille génique des *lipid transfer*/*lipopolysaccharide binding proteins* (LT/LBP) qui comprend également la *cholesteryl ester transfer protein* (CETP), la *lipopolysaccharide binding protein* (LBP) et la *bactericidal permeability increasing protein* (BPI).

L'ADNc de la PLTP humaine, qui a été isolé à partir de cellules endothéliales, a une longueur de 1750 pb, et contient un cadre ouvert de lecture de 1518 pb, codant pour une protéine mature de 476 acides aminés, précédée par une séquence-signal de 17 acides aminés ; la séquence de la PLTP mature contient, outre de nombreux sites potentiels de O-glycosylation, 6 sites potentiels de N-glycosylation aux acides aminés 47, 77, 100, 126, 228, et 381 (DAY et al., J Biol Chem, 269, 9388-91, 1994). Il a été montré que la PLTP portait effectivement des structures N-glycanniques complexes, et que la N-glycosylation jouait un rôle essentiel dans la sécrétion de la protéine, et participait également à son activité biologique (HUUSKONEN & EHNHOLM, Curr Opin Lipidol, 11, 285-9, 2000; QU et al., Protein J, 25, 167-73, 2006).

Il a été observé que la PLTP existe dans le plasma sous 2 formes, l'une active et l'autre inactive (OKA et al., J Lipid Res, 41, 1651-7, 2000), associées à 2 types différents de particules de lipoprotéines. La forme active est associée à des particules ayant un poids moléculaire moyen de 160 kDa, voisin de celui des HDL (*high density lipoproteins*) tandis que la forme inactive est associée à des particules ayant un poids moléculaire moyen de 520 kDa, intermédiaire entre celui des HDL et des LDL (*low density lipoproteins*). KARKKAINEN et al. (J Biol Chem, 277, 15413-8, 2002) décrivent la séparation de ces 2 formes à partir du plasma, et rapportent que la forme active est associée à l'apolipoprotéine E, et la forme inactive à l'apolipoprotéine A-I. D'autres auteurs rapportent au contraire que dans le plasma, la forme active est associée principalement à l'apolipoprotéine A-I, et la forme inactive à l'apolipoprotéine E (CHEUNG & ALBERS, J Lipid Res, 47, 1315-21, 2006).

Outre son activité de transfert des phospholipides, la PLTP permet également le transfert d'autres molécules amphiphiles, notamment des lipopolysaccharides (LPS) bactériens vers des lipoprotéines sériques. Des expérimentations *in vitro* ont montré que de la PLTP recombinante exprimée dans des surnageants de cultures cellulaires pouvait transférer des LPS des parois de bactéries gram-négatives vers des particules HDL, et neutraliser les effets biologiques de ces LPS, en prévenant leur liaison aux récepteurs des membranes cellulaires, notamment CD14 et TLR4 (HAILMAN et al., J Biol Chem, 271, 12172-8, 1996) (VESY et al., Infect Immun, 68, 2410-7, 2000).

Cette propriété de la PLTP présente un intérêt tout particulier, car les LPS bactériens ont des effets pro-inflammatoires très puissants. Leur interaction avec les récepteurs cellulaires induit la production de cytokines proinflammatoires, et le déclenchement d'une très forte réponse inflammatoire dont la manifestation la plus grave, le choc endotoxinique, ou choc septique, peut entrainer la mort. Il a été suggéré, sur la base des propriétés de transfert et de neutralisation des LPS observées *in vitro,* que la PLTP pouvait être utilisée pour le traitement du choc septique (Brevet US 5,932,536).

Le rôle joué par la PLTP dans la neutralisation des effets induits par les LPS a été indirectement confirmé *in vivo,* chez des souris knock-out pour le gène de la PLTP, et n'exprimant donc pas cette protéine (souris PLTP-KO). Il a été observé que chez ces animaux, après une injection unique de LPS, l'association des LPS avec les lipoprotéines circulantes était retardée, la production de cytokines inflammatoires (IFN-gamma, IL-6, TNF-alpha) augmentée et la durée de survie était plus courte que chez les souris de type-sauvage (GAUTIER et al., J Biol Chem, 283, 18702-10, 2008).

Toutefois, les propriétés biologiques de la PLTP et ses éventuelles utilisations thérapeutiques n'avaient jusqu'à présent pas pu être vérifiées par des expérimentations d'administration de PLTP *in vivo,* car on ne disposait d'aucune préparation de PLTP utilisable dans ce cadre.

Initialement, la PLTP a été purifiée à partir du plasma humain (DAY et al., J Biol Chem, 269, 9388-91, 1994; TOLLEFSON et al., J Lipid Res, 29, 1593-602, 1988; LAGROST et al., J Lipid Res, 35, 825-35, 1994). Cependant le plasma est relativement pauvre en PLTP (de l'ordre de 50 microgrammes de PLTP par gramme de protéines plasmatiques totales), et s'agissant en outre d'un milieu complexe, la purification de PLTP à partir de ce milieu nécessite une succession d'étapes chromatographiques lourdes à mettre en oeuvre, et aboutissant à un taux de rendement de purification très bas (de l'ordre du 1%). En outre, l'activité de la PLTP plasmatique peut varier selon son association aux lipoprotéines plasmatiques et elle apparait généralement, après purification, sous la forme de plusieurs bandes distinctes, dont la nature et l'activité restent incertaines.

Plusieurs tentatives pour produire de la PLTP recombinante de différentes espèces de mammifères dans divers types de cellules eucaryotes transfectées ont été effectuées : des cellules BHK ont été utilisées pour la production de PLTP humaine ou murine (ALBERS et al., Biochim Biophys Acta, 1258, 27-34, 1995) ; des cellules COS-1 pour la production de PLTP de porc (PUSSINEN et al., J. Lipid Res., 38, 1473-81, 1997) ; des cellules d'insecte Sf-9 infectées par un baculovirus (HUUSKONEN et al., Biochim Biophys Acta, 1391, 181-92, 1998) ; des cellules HeLa (HUUSKONEN et al., J. Lipid Res., 39, 2021-30, 1998). Ces systèmes d'expression ont conduit à la production de protéines de poids moléculaire variable, apparaissant souvent sous la forme de bandes multiples. Il a été proposé, pour produire de la PLTP recombinante se rapprochant le plus possible de la PLTP plasmatique, d'utiliser des cellules hépatocytaires HepG2, déjà couramment utilisées pour étudier la sécrétion de diverses lipoprotéines et apolipoprotéines ; en effet, il a été supposé que la majeure partie de la PLTP plasmatique étant produite par le foie, ces cellules pouvaient fournir un environnement cellulaire optimal pour reproduire les caractéristiques de la PLTP native (GUO et al., J Nutr, 129, 1984-91, 1999). Effectivement, l'utilisation de ce système d'expression a permis d'obtenir une PLTP recombinante possédant des caractéristiques similaires à celle de la protéine native ; la purification de cette PLTP recombinante a ensuite montré qu'elle était associée à l'apolipoprotéine E produite par les cellules HepG2, dans des particules de lipoprotéines de 160 kDa, ressemblant à la forme active de la PLTP plasmatique précédemment décrite par OKA *et al.* et KARKKAINEN *et al.* (2000, 2002, précités).

Cependant, ce système d'expression, comme ceux précédemment décrits, ne permet pas la production de quantités suffisantes de PLTP pour permettre de tester son activité *in vivo,* et encore moins de l'utiliser à des fins thérapeutiques.

Il est donc souhaitable de disposer d'une source de PLTP recombinante permettant de produire cette protéine sous forme active et en quantité suffisante pour envisager son utilisation effective.

Une alternative à la production de protéines d'intérêt thérapeutique par des cultures de cellules transformées est leur production par des animaux transgéniques, et notamment dans le lait de ceux-ci (HOUDEBINE, "Production of pharmaceutical proteins by transgenic animals. Comparative Immunology", Microbiology & Infectious Diseases, 32 : 107-121, 2009). Cette approche présente théoriquement de nombreux avantages, et notamment un rendement plus élevé, un coût de production plus faible que la production en cultures de cellules, et une montée en puissance de la production plus aisée et plus flexible au niveau industriel. Cependant, bien que la possibilité de produire dans du lait de souris transgéniques une protéine recombinante, l'inhibiteur de plasminogène (tPA), possédant des propriétés biologiques similaires à celles de la protéine humaine native ait été démontrée il y a de nombreuses années (GORDON et al., Bio Technol, 5, 1183-7, 1987), le nombre de protéines qui ont pu être produites avec succès dans le lait d'animaux transgéniques demeure encore relativement limité. En effet, de nombreux facteurs, dépendant notamment de la nature de la protéine d'intérêt à exprimer peuvent conditionner le succès ou l'échec.

Parmi ces facteurs figure notamment la capacité de l'hôte à produire, dans les glandes mammaires, des protéines présentant des modifications post-traductionnelles similaires à celles de la protéine humaine native. Ce facteur est particulièrement critique si l'on envisage de produire sous forme recombinante dans le lait des protéines comme la PLTP, dont les modifications post-traductionnelles sont multiples et variées (clivage du peptide signal et du propeptide, O-glycosylation et N-glycosylation), et qui sont naturellement produites dans le foie. En effet, les cellules de la glande mammaire ont des capacités de modifications post-traductionnelles différentes de celles des cellules hépatiques.

Il a par exemple été observé, dans le cas de facteurs de coagulation, qui font partie des protéines dont la production dans le lait d'animaux transgéniques a été le plus étudiée, que des souris transgéniques exprimant la protéine C humaine dans leurs glandes mammaires ne pouvaient pas effectuer correctement le clivage du propeptide et la γ-carboxylation (DROHAN et al., Transgenic Res., 355-64, 1994). Des observations similaires ont été effectués chez des porcs transgéniques, pour la protéine C et pour le facteur IX (LEE et al., J. Biochem., 118, 81-7, 1995 ; VAN COTT et al., Genet Anal., 15, 155-60, 1999).

Un autre facteur important est l'environnement constitué par le lait. Il s'agit d'un milieu hétérogène complexe, très riche en protéines et en lipides : il se présente sous forme d'une émulsion de globules gras dans un liquide qui est lui-même une suspension colloïdale de micelles de caséine dans une phase aqueuse, le lactosérum.

Le lactosérum contient notamment du lactose, des sels minéraux, ainsi que des vitamines et des protéines hydrosolubles. Les micelles de caséine sont des structures supramoléculaires constituées de différents types de caséines (alpha, beta, kappa) qui sont phosphorylées à des degrés variables et associées au calcium via les groupes phosphates. La majeure partie des lipides est dispersée dans le lait sous forme de globules sphériques enrobées d'une membrane lipoprotéique (DANTHINE et al., Lait, 80, 209-22, 2000), la « membrane du globule gras du lait » ou « *milk fat globule membrane* » (MFGM), qui est un assemblage complexe principalement constitué de protéines diverses (MATHER, J. Dairy Sci., 83, 203-47, 2000), associées à des phospholipides et des lipides neutres.

Compte tenu de la nature très lipophile de la PLTP et de sa capacité à lier le cholestérol, les phospholipides et les diacylglycérides, il pourrait être supposé que si elle est produite dans le lait sous forme recombinante, elle s'associera préférentiellement avec les membranes des globules gras. Dans la mesure où il est par ailleurs connu que l'activité de la PLTP dépend du type de structure lipoprotéique avec laquelle elle est associée, les effets d'une interaction entre la PLTP et les globules gras du lait apparaissent difficilement prévisibles.

Par ailleurs, la purification d'une protéine recombinante d'intérêt produite dans le lait d'un animal transgénique débute classiquement par une étape de clarification du lait, destinée à éliminer les micelles de caséine (par exemple par précipitation à pH acide, suivie de centrifugation), et les globules gras (par exemple par centrifugation). La protéine d'intérêt est ensuite purifiée à partir du lactosérum. Cette approche est généralement efficace dans le cas de protéines hydrophiles. Cependant, si la protéine est capturée par les micelles de caséines ou par les globules gras, sa purification peut nécessiter l'utilisation de méthodes beaucoup plus drastiques, risquant d'être délétères pour son activité biologique.

Or, les Inventeurs ont maintenant trouvé que, de façon surprenante, des lapines transgéniques exprimant dans leurs glandes mammaires une séquence codant pour la PLTP humaine produisaient dans leur lait une PLTP recombinante active, et qu'en outre cette PLTP pouvait être extraite à partir du lactosérum.

La présente invention a en conséquence pour objet une lapine transgénique exprimant de la PLTP humaine active dans son lait.

Un animal transgénique conforme à l'invention contient dans son génome une ou plusieurs copies d'un transgène comprenant un polynucléotide codant pour la PLTP humaine, placé sous contrôle transcriptionnel d'un promoteur permettant son expression spécifique dans les cellules des glandes mammaires dudit animal.

On entend par « transgène » une construction d'acide nucléique insérée de façon stable dans le génome d'un organisme hôte, qui se transmet à sa descendance de génération en génération. Dans le cas présent, le transgène permet l'expression d'une protéine d'intérêt (la PLTP) dans le lait de l'animal-hôte transgénique.

Le promoteur utilisé pour exprimer la PLTP peut être un promoteur endogène d'un gène exprimé dans les glandes mammaires de l'organisme hôte, ou un promoteur exogène. Des promoteurs permettant l'expression spécifique dans les cellules de la glande mammaire sont connus en eux-mêmes. Il peut s'agir par exemple des promoteurs de gènes de caséines ou de protéines sériques du lait : on citera notamment les promoteurs des caséines α, β, ou κ, le promoteur de la β-lactoglobine, le promoteur de l'α-lactalbumine, celui de la WAP (« *Whey Acidic Protein* »), ou celui de la lactoferrine. Un promoteur préféré est celui de la WAP, décrit dans la Demande EP 0 527 063. On peut avantageusement utiliser une séquence d'ADN optimisée pour l'expression dans les glandes mammaires de l'animal-hôte utilisé. Une telle séquence peut être obtenue *in silico* par des techniques bien connues par l'homme de l'art, afin d'éliminer les sites cryptiques d'épissage, les séquences riches en A/T, déstabilisatrices des ARNm, les sites de polyadenylation ainsi que les boites TATA potentiellement parasites, et les ilots CpG, et d'optimiser les codons pour refléter les préférences des cellules de la glande mammaire de l'animal-hôte pour produire les protéines du lait.

Avantageusement, le transgène contient en outre d'autres éléments destinés à optimiser la transcription et/ou la traduction de la protéine recombinante. De tels éléments sont connus en eux-mêmes de l'homme de l'art (cf. par exemple HOUDEBINE, « Design of expression cassettes for the génération of transgenic animals (including insulators) », Rat Genomics: Methods in Molecular Biology, Vol 597 : 55-69, 2009).

Il peut s'agir notamment :
- d'un isolateur fort, placé en 5' du promoteur, garantissant un niveau d'expression de la protéine dépendant en partie du nombre de copies de transgène intégrées et dépendant moins du site d'intégration du transgène dans le génome de l'animal : on citera par exemple la région 5'HS4 du gène de la béta-globine de poulet (TABOIT-DAMERON et al., Transgenic. Res., 8 : 223-235, 1999; RIVAL-GERVIER et al., Transgenic. Res., 12 : 723-730, 2003).
- d'un ou plusieurs couples exon/intron pouvant contenir un ou plusieurs amplificateurs (*enhancer*) de transcription ou de traduction : à titre d'exemples, on peut citer : les introns des gènes tardif et précoce du génome du virus SV40, le deuxième intron d'un gène de beta-globine, les introns du gène EF1 alpha, les introns du gène de l'alpha-s1 caséine, les introns du gène WAP, les introns des gènes de l'hormone de croissance humaine et bovine ; les séquences « enhancer » peuvent notamment être choisies parmi celles présentes dans les séquences LTR du virus HTLV, ou du virus MMTV (virus de la tumeur mammaire murine), la séquence « enhancer » du gène des immunoglobines, la séquence « enhancer » du gène de l'alpha s-1 caséine, la séquence « enhancer » de la béta-globine. La séquence « enhancer » peut également être la région distale en amont (jusqu'à 140 kpb) du gène WAP ou la région distale en aval (au moins 10 kpb) du gène WAP, telles qu'elles sont décrites par RIVAL-GERVIER et al. (Mol. Reprod. Develop., 63 : 161-167, 2002), ou dans la Demande EP 1 217 071.
- un terminateur fort garantissant une terminaison efficace de la transcription : à titre d'exemples, on citera les terminateurs des gènes précoce ou tardif du virus SV40, ceux des gènes de béta-globine, des gènes WAP, des gènes de l'hormone de croissance humaine ou bovine.

La transgénèse peut s'effectuer par les méthodes classiques connues en elles-mêmes. Avantageusement, la construction de gène est introduite par microinjection dans l'un des pronuclei d'embryons fécondés qui sont ensuite réimplantés chez des femelles porteuses. L'obtention des animaux transgéniques est également possible par clonage par transfert de noyau suivi par transfert d'embryon dans des femelles receveuses ; ou par ICSI *(Intra Cytoplasmic Sperm Injection),* les spermatozoïdes étant incubés en présence d'un transgène, puis introduits dans un ovocyte ; ou encore par la chimérisation d'embryons à partir de cellules génétiquement modifiées *in vitro,* utilisée notamment chez le rat et chez la souris.

La présente invention a également pour objet le lait produit par les animaux transgéniques conformes à l'invention, et l'utilisation de ce lait en tant que matière première pour la préparation de PLTP humaine recombinante.

La présente invention a aussi pour objet un procédé d'obtention d'une préparation de PLTP humaine recombinante à partir du lait d'un animal transgénique conforme à l'invention, caractérisé en ce qu'il comprend la clarification du lait, et la récupération du lactosérum.

La clarification du lait peut s'effectuer par des méthodes classiques connues en elles-mêmes de l'homme du métier. On citera notamment la précipitation des caséines à pH acide (inférieur ou égal à 4,6), éventuellement en présence d'un agent chélatant, suivie de centrifugation pour éliminer d'une part les globules gras d'autre part le précipité de caséine. Le lait peut également être clarifié par ultracentrifugation ou par filtration tangentielle.

Le lactosérum ainsi récupéré constitue une préparation de PLTP recombinante, et peut être utilisé tel quel, éventuellement après concentration, dans certaines applications.

Toutefois, on préférera généralement effectuer une étape supplémentaire d'extraction de la PLTP à partir de ce lactosérum, permettant d'obtenir une préparation enrichie en PLTP.

Avantageusement, cette extraction de la PLTP à partir du lactosérum est effectuée par chromatographie d'affinité sur héparine liée à un support solide approprié.

La chromatographie d'affinité sur héparine est effectuée de manière classique en chargeant le lactosérum sur une colonne d'héparine liée à un support solide approprié, en effectuant l'élution à force ionique croissante, et en récupérant les fractions contenant l'activité de transfert des phospholipides.

Généralement la chromatographie sera effectuée au pH physiologique, soit généralement à un pH d'environ 7,4, en présence de concentrations croissantes de NaCl, de 0 à 1 mM. Les fractions contenant l'activité de transfert des phospholipides sont éluées dans une plage de concentration en NaCl de 250 à 450 mM, de préférence de 300 à 400 mM.

Le procédé de la présente invention permet d'obtenir une préparation de PLTP humaine recombinante.

Cette préparation peut être utilisée dans toutes les applications proposées pour la PLTP humaine, et notamment pour la prévention ou le traitement du choc septique.

Avantageusement, cette préparation est formulée sous forme d'une composition administrable par voie parentérale, de préférence par voie injectable.

La présente invention sera mieux comprise à l'aide du complément de description qui va suivre, qui se réfère à des exemples non-limitatifs, illustrant la production de PLTP humaine recombinante à partir du lait de lapines transgéniques, ainsi que son efficacité dans le traitement du choc septique.

### LÉGENDE DES FIGURES :

**Figure 1** **:** Comparaison des activités de transfert des phospholipides dans les laits de lapines WT (Milk A et Milk B) et PLTPTg_{WAP} (Milk 04, Milk 06 et Milk 13). En abscisse : temps en minutes ; en ordonnée : activité de transfert des phospholipides (en unités de fluorescence).
**Figure 2** **:** Profil FPLC d'élution des protéines de lait clarifié sur colonne d'Héparine Sépharose HR26/16, en utilisant un gradient discontinu de NaCl avec incréments de 50 mM. En abscisse : volume d'élution ; en ordonnée : absorbance à 280 nm.
**Figure 3** **:** Electrophorèse sur gel de polyacrylamide en gradient en conditions dénaturantes des fractions #167, #175 et #178 éluées sur colonne d'Héparine Sépharose.
**Figure 4** **:** Profil FPLC d'élution des protéines de lait clarifié sur colonne d'Héparine Sépharose HR26/16, en utilisant un gradient discontinu de NaCl avec incréments de 100 mM. En abscisse : volume d'élution en millilitres ; en ordonnée : absorbance à 280 nm.
**Figure 5** **:** Electrophorèse sur gel de polyacrylamide en gradient en conditions dénaturantes de la fraction #68 éluée sur colonne d'Héparine Sépharose et présentant une activité spécifique de transfert des phospholipides élevée. « unbound » : fractions non liées ; « bound » : fractions liées.
**Figure 6** **:** Second run d'électrophorèse (Idem Figure 5 avec temps plus court de coloration au Bleu de Commassie). « bound » : fractions liées.
**Figure 7** **:** Activité de transfert des phospholipides dans des plasmas de souris PLTP-KO injectées soit avec la fraction de PLTP purifiée (barres noires), soit avec un volume identique de tampon PBS (barres blanches). En abscisse : les différents temps de prélèvement ; en ordonnée : activité de transfert des phospholipides (en unités de fluorescence).
**Figure 8** **:** Concentrations plasmatiques de cytokines après injection de LPS, chez des animaux ayant reçu de la PLTP recombinante (barres noires), ou du tampon PBS (barres blanches).
**Figure 9** **:** Aire sous la courbe des concentrations de cytokines mesurées sur une période de 24 heures et exprimées en pg/ml de plasma x heure.
**Figure 10** **:** Activité de transfert des phospholipides des plasmas de souris PLTP-KO ayant reçu 15mg/kg de LPS, et injectées soit avec la fraction de PLTP purifiée (barres noires), soit avec un volume identique de tampon PBS (barres blanches).
**Figure 11** **:** Courbes de Kaplan-Meier montrant la mortalité des souris PLTP-KO ayant reçu une dose unique de LPS (15 mg/kg, i.p.). Flèches : plusieurs injections intraveineuses (veine caudale) de tampon (PBS) ou de PLTP recombinante (PLTP) aux temps 15 min, 1h, 2h, 3h, 4h, 5h et 7h post LPS.
**Figure 12** **:** Courbes de Kaplan-Meier montrant la mortalité des souris PLTP-KO ayant reçu une dose unique de LPS (25 mg/kg, i.p.). Flèches : une injection unique de LPS puis plusieurs injections intraveineuses (veine caudale) de PLTP recombinante active (PLTP active) ou de PLTP recombinante inactivée (PLTP inactivée) aux temps 1h, 5h, 10h, 24h, 32h, 48h, 56h, 72h et 80h post LPS.
**Figure 13** **:** Comparaison des activités de transfert des phospholipides de la PLTP recombinante active, de la PLTP recombinante inactivée (2h, 65°C), d'un plasma de souris wild-type (WT), d'un plasma de souris PLTP-KO, et d'un tampon PBS. En abscisse : temps en minutes ; en ordonnée : activité de transfert des phospholipides (en unités de fluorescence).

### EXEMPLE 1: CONSTRUCTION D'UN VECTEUR DE TRANSGÉNÈSE CONTENANT LA SÉQUENCE CODANT POUR LA PLTP HUMAINE

### Clonage de l'ADNc de la PLTP dans un vecteur intermédiaire

Une séquence d'ADNc synthétique rétrotraduit codant pour le précurseur de la PLTP humaine (UniProtKB/Swiss-Prot P55058), bordée, en position 5', d'une séquence Kozak consensus et d'un site de restriction unique *Mlu*I et, en position 3', de deux codons stop et d'un site de restriction *Nhe*I, a été synthétisée et clonée dans un vecteur intermédiaire, dérivé du plasmide pBluescript, contenant le gène de résistance à l'ampicilline ainsi que l'origine de réplication bactérienne Col E1.

### Vecteur de transgénèse

Le vecteur de transgénèse utilisé pour le clonage est dérivé du plasmide pPolyIII, possédant un gène de résistance à l'ampicilline ainsi que l'origine de réplication bactérienne Col E1. Ce vecteur de transgénèse contient une cassette d'expression comprenant : un dimère de la séquence de l'isolateur 5'HS4 du gène de la béta-globine de poulet (Genbank U78775) (RECILLAS-TARGA et al., Proc. Natl. Acad. Sci., 10 : 6883-6888, 2002) en amont du promoteur WAP (*whey acidic protein* ; Genbank X52564) de lapin de 6,3 kpb (RIVAL-GERVIER et al., Transgenic Res., 6 :723-730, 2003), le deuxième intron du gène de beta-globine de lapin (Genbank V00882) contenant un amplificateur de transcription ; un second amplificateur de transcription (SUR 1.2.3) contenant la séquence 5'UTR des gènes précoces de SV40 fusionnée avec la région R et le début de la région U5 de HTLV-1 (ATTAL et al., FEBS Lett., 392, 220-224, 1996); un troisième amplificateur de transcription (Igµ2), dérivé de la région mu de la chaine lourde d'IgG de souris (Genbank J00440) (GILLIES et al., Cell 33, 717-728, 1983) et le terminateur de transcription de l'hormone de croissance humaine (Genbank M13438). Cette cassette contient un site *Mlu*I et un site *Nhe*I localisés entre le second et le troisième amplificateur de transcription ; elle est flanquée de part et d'autres de sites *Not*I permettant l'excision des séquences du plasmide pPolyIII.

L'insert d'ADN codant pour la PLTP, récupéré par digestion *Mlu*I/*Nhe*I à partir du vecteur intermédiaire a été inséré entre les sites *Mlu*I et *Nhe*I de la cassette d'expression.

Le vecteur de transgénèse résultant contient un insert qui est constitué dans son orientation 5' vers 3' par i) le dimère de la séquence de l'isolateur 5'HS4 du gène de la béta-globine de poulet, ii) le promoteur WAP (*whey acidic protein*) de lapin de 6,3 kpb, iii) l'intron contenant le premier amplificateur de transcription, iv) le second amplificateur de transcription, v) l'ADNc de la PLTP humaine, vi) le troisième amplificateur de transcription, et vii) le terminateur de transcription.

Comme précédemment, les colonies contenant le vecteur recombinant sont sélectionnées sur la base de leur résistance à l'ampicilline, puis la présence de l'insert est contrôlée par analyse des fragments de restrictions, puis par séquençage.

### EXEMPLE 2: PRODUCTION DE LAPINES TRANSGÉNIQUES EXPRIMANT LA PLTP HUMAINE DANS LEURS GLANDES MAMMAIRES

Les lapines transgéniques ont été obtenues par la technique classique de micro-injection (BRINSTER et al., Proc. Natl. Acad. Sci., 82 : 4438-4442, 1985).

### Préparation des inserts pour la transgenèse

Le vecteur de transgènese contenant la séquence codant pour la PLTP recombinante a été digéré par l'enzyme de restriction *Not*I et l'insert contenant le transgène a été isolé sur gel d'agarose puis purifié sur ElutipD (Schleicher-Schuell, Ecquevilly, France) conformément aux instructions du fabricant, précipité à l'éthanol puis repris dans un tampon 10 mM Tris-HCl, 0,1 mM EDTA, pH 7,4.

### Préparation des lapines donneuses et receveuses

Des lapines Néo-zélandaises donneuses d'embryons, âgées de 16-30 semaines, sont traitées par voie sous-cutanée pendant 3 jours par de la FSH porcine (Follicular Stimulating Hormone) pour stimuler le développement folliculaire. Au troisième jour, les lapines reçoivent une injection intraveineuse d'hormone hCG (human Choroidic Hormone), puis les femelles sont accouplées.

Les lapines receveuses sont âgées de 18-20 semaines. Une pseudogestation synchronisée est induite soit en gardant les femelles une semaine en cycle de jours longs (16 h de lumière) avant de les accoupler avec des mâles vasectomisés, soit par un traitement hormonal (superovulation FSH/LH).

### Micro-injection des embryons prélevés et implantation

Au 4ème jour, 18-19h après l'accouplement, les embryons sont prélevés sur les lapines donneuses pour procéder à la micro-injection d'ADN : la micro-injection d'ADN se déroule juste après les prélèvements (15-25 h après l'accouplement). Les embryons au stade une cellule sont placés dans une micro-goutte de milieu sous un microscope inversé équipé d'objectifs Normarsky et de micro-manipulateurs. Des embryons individuels sont positionnés et sécurisés en utilisant une pipette. Le transgène dilué à une concentration pouvant varier de 1ng/µl à 6ng/µl, dans du tampon 10 mM Tris-HCl, 0.1 mM EDTA, pH 7,4 est micro-injecté préférentiellement dans le pronucléus mâle de l'embryon à l'aide d'une pipette d'injection.

Suite à la micro-injection, les embryons sont maintenus en culture *in vitro* pendant 1 à 5 h (35 à 40°C, 3 à 5% CO₂ dans l'air). La qualité des embryons micro-injectés est alors rapidement évaluée sous stéréomicroscope. Les embryons unicellulaires intacts sont alors réimplantés sous anesthésie générale dans le lumen des oviductes des lapines receveuses synchronisées (10 embryons dans chaque oviducte), en utilisant une procédure chirurgicale (les oviductes sont extériorisés par laparotomie). La parturition peut intervenir naturellement 29-31 jours après le transfert d'embryons. Si nécessaire, on procède au déclenchement par injection d'ocytocine le 31ème jour. Le nombre de lapereaux nés par rapport au nombre d'embryons réimplantés est de l'ordre de 5 à 20%.

### EXEMPLE 3: SÉLECTION ET CARACTÉRISATION DES LAPINS TRANSGÉNIQUES

Pendant le processus d'embryogenèse, l'ADN recombinant micro-injecté intègre le génome de manière aléatoire. Les lapins nouveau-nés (10 jours) sont testés pour la présence du transgène par une biopsie de l'oreille. L'ADN génomique est extrait et une analyse PCR (Polymerase Chain Reaction) est réalisée en utilisant des amorces spécifiques de l'insert recombinant.

Les lapins pour lesquels le transgène a été mis en évidence sont appelés "Fondateurs F0".

Les lignées des fondateurs F0 ont été en outre caractérisées par i) analyse du nombre de copies de transgène intégrées dans leur génome, et ii) détermination du nombre de sites d'intégration.

Le nombre de copies du transgène intégrées dans le génome de chaque lignée de fondateur F0 a été déterminé par PCR quantitative et par transfert de Southern. Ce nombre varie, selon la lignée, de 1 copie par cellule à une centaine de copies par cellule.

Le nombre de sites d'intégration a également été déterminé par transfert de Southern. Ce nombre varie, selon la lignée, de 1 à 3 sites par génome.

### EXEMPLE 4: EVALUATION DE L'EXPRESSION DE PLTP HUMAINE RECOMBINANTE DANS LE LAIT DES LAPINES TRANSGÉNIQUES

L'activité de transfert des phospholipides dans le lait de lapines transgéniques F0 de l'Exemple 3 a été évaluée.

Pour ce faire, l'activité de transfert des phospholipides par la PLTP a été mesurée dans les échantillons de lait à l'aide du kit commercial de chez Roar Biomedical Inc. (New York, NY, USA) selon les recommandations du fabricant. Un phospholipide fluorescent, en l'occurrence la phosphatidylcholine, est présent dans un état d'auto-extinction lorsqu'il est associé avec un substrat donneur de type liposome. L'activité de transfert des phospholipides de la PLTP est déterminée par l'augmentation de l'intensité de fluorescence lorsque le phospholipide fluorescent est transféré vers un substrat lipoprotéique accepteur, par exemple de type VLDL (« *very low density lipoprotein* »), présent dans le milieu d'incubation (Masson D. et al. Mol Human Reprod 2003; 9:457).

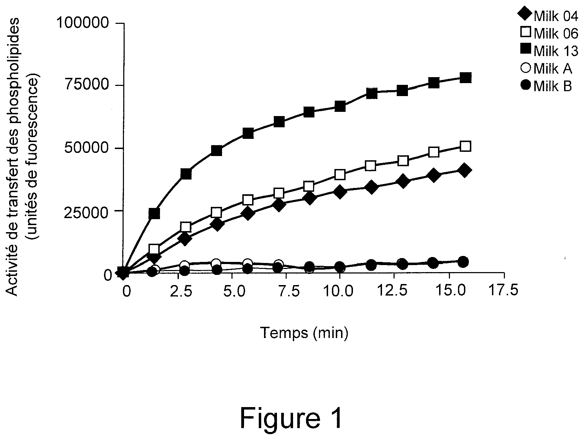

La Figure 1 représente la comparaison entre l'activité de transfert des phospholipides dans les laits de lapines non transgéniques, ou lapines WT (Milk A et Milk B), et les laits de plusieurs lignées de lapines transgéniques pour la PLTP, ou lapines PLTPTg_{WAP} (Milk 04, Milk 06 et Milk 13). Alors qu'aucune activité significative de transfert des phospholipides n'est détectée dans les laits de lapines WT, les laits de lapines PLTPTg_{WAP} montrent une capacité temps-dépendante à échanger des phospholipides fluorescents entre des liposomes synthétiques et des lipoprotéines VLDL plasmatiques. Le lait Milk 13, qui présente l'activité de transfert des phospholipides la plus élevée, a été utilisé pour la purification de la protéine.

### EXEMPLE 5: OBTENTION D'UNE PRÉPARATION DE PLTP HUMAINE ACTIVE À PARTIR DU LAIT DE LAPINES TRANSGÉNIQUES

### 1 - Précipitation des caséines à pH acide et température ambiante

1 volume de lait est mélangé avec 2 volumes d'EDTA 0,5 mM, pH 8,0 et 7 volumes d'eau MilliQ. L'EDTA permet de chélater les ions calcium et de casser les micelles de caséine, susceptibles de retenir une partie de la PLTP.

Le pH est abaissé progressivement jusqu'à 4 par ajout d'acide acétique glacial en goutte à goutte sous agitation, afin de précipiter totalement les caséines (dont le pHᵢ est de 4,6).

### 2 - Clarification du petit lait par centrifugation basse vitesse et neutralisation du pH

Après précipitation des caséines à pH acide, centrifugation du mélange pendant 10 min à 2000g et à 4°C.

Recueil de la fraction clarifiée intermédiaire située entre le surnageant lipidique et le culot de protéines.

Neutralisation avec Tris solide en quantité suffisante pour l'obtention du pH 7,4.

Filtration de la fraction ainsi recueillie sur filtre en fibre de verre (Millipore AP2004700). Il s'agit d'une filtration classique, avant injection sur colonne. Elle permet d'éliminer les agrégats et/ou particules de grande taille et de protéger ainsi la colonne en évitant son colmatage par ces agrégats et/ou particules de grande taille.

### 3 - Extraction/purification de la PLTP par chromatographie d'affinité sur colonne d'Héparine Sépharose

Injection de la fraction protéique, clarifiée et filtrée, à température ambiante sur colonne d'Héparine Sépharose 6 Fast Flow (240x16 mm ID) préalablement équilibrée avec un tampon 20 mM Tris, pH 7,4.

Débit d'injection : 1 ml/min avec pompe péristaltique.

Rinçage de la colonne une nuit avec un tampon Tris 20 mM/pH 7,4.

Branchement de la colonne chargée sur système FPLC Äkta.

Programmation d'un gradient discontinu du tampon Tris 20 mM/ pH 7,4 vers le tampon Tris 20mM/NaCl 1 M/pH 7,4 (avec incréments de 50 ou 100 mM NaCl).

Dialyse de la fraction contre un tampon PBS 150 mmol/l NaCl, pH 7,4

L'activité de transfert des phospholipides est mesurée dans chacune des fractions (selon le même protocole que dans l'exemple 4), et rapportée aux valeurs de dosage BCA des protéines (dosage avec un kit commercial Bicinchoninic Acid Assay, Interchim, Montluçon, France).

### Résultats

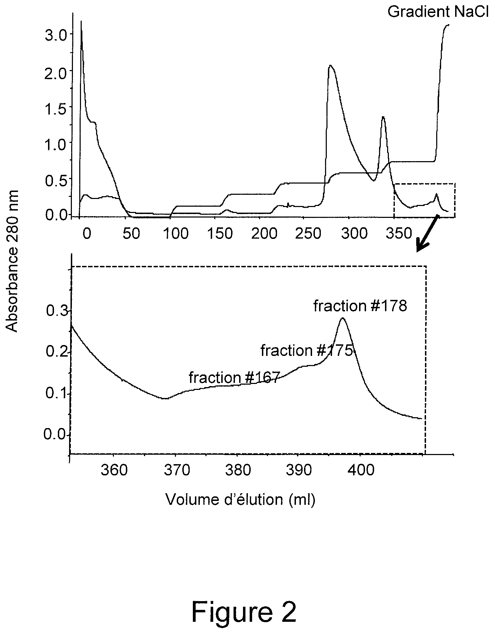

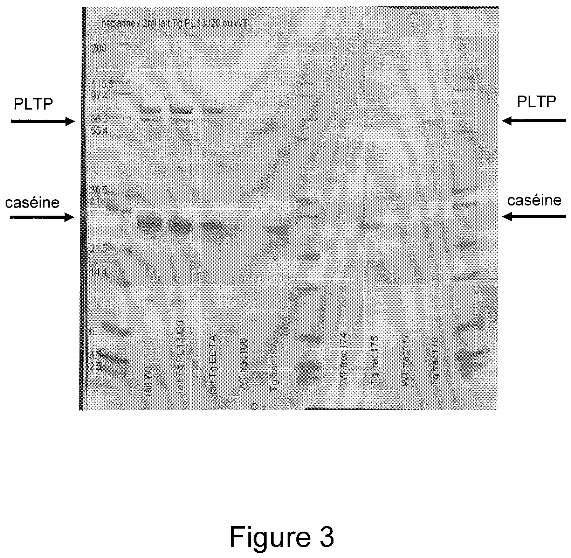

Les résultats d'une extraction effectuée en utilisant un gradient discontinu de NaCl avec incréments de 50 mM sont illustrés par les Figures 2 et 3.

Comme le montre la Figure 2, les protéines de la fraction écrémée de lait sont éluées de la colonne d'héparine en plusieurs pics successifs selon le gradient discontinu de NaCl. Plusieurs pics discrets (fractions #167, #175 et #178) sont élués aux plus fortes concentrations de NaCl (supérieures à 250 mM).

Les résultats de la mesure d'activité de transfert des phospholipides sont résumés dans le Tableau I ci-dessous.

**TABLEAU I**

| | [prot] g/l | Activité | Activité spécifique (AS) | Rapport AS fraction/ AS lait Tg |
|---|---|---|---|---|
| Lait Tg | 92,73 | 17781 | 192 | 1 |
| Fraction #61 | 0,185 | 47 | 254 | 1 |
| Fraction #91 | 0,163 | 379 | 2325 | 12 |
| Fraction #102 | 0,185 | 496 | 2681 | 14 |
| Fraction #119 | 12,398 | 4119 | 332 | 2 |
| Fraction #149 | 5,44 | 5750 | 1057 | 6 |
| Fraction #167 | 0,215 | 3262 | 15172 | 79 |
| Fraction #175 | 0,21 | 2256 | 10743 | 56 |
| Fraction #178 | 0,469 | 4715 | 10053 | 52 |

L'activité spécifique de transfert des phospholipides (colonne « Activité spécifique (AS) » du Tableau I) est la plus forte dans les fractions #167, #175 et #178, où elle est plus de 50 fois plus élevée que dans le lait de départ (dernière colonne de droite du Tableau I).

La Figure 3 montre que ces fractions sont constituées de 2 protéines majoritaires, présentant respectivement un poids moléculaire apparent de 30 et 60 kDa. La bande 30 kDa correspond aux caséines, retrouvées en abondance dans les laits WT et PLTPTg_{WAP}. La bande 60 kDa se trouve dans la zone des PM classiquement rapportés pour la PLTP (compris entre 50 et 70 kDa).

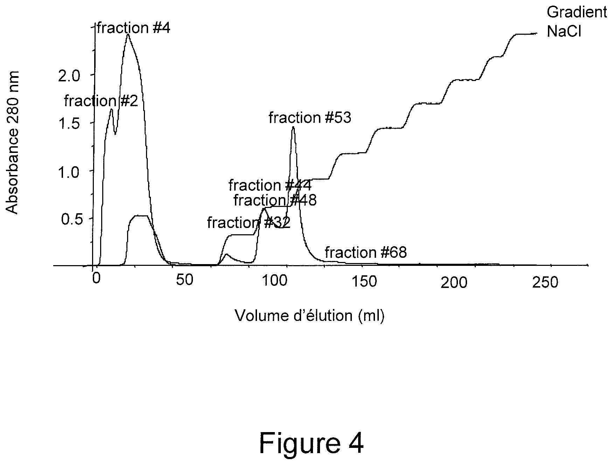

Les résultats d'une extraction effectuée en utilisant un gradient discontinu de NaCl avec incréments de 100 mM sont illustrés par les Figures 4 à 6, ainsi que le Tableau II ci-dessous.

La fraction présentant l'activité de transfert des phospholipides spécifique la plus élevée (fraction #68) est éluée au palier 300 mM de NaCl (Figure 4).

Cette fraction a une activité spécifique 44 fois plus élevée que le lait de départ (dernière colonne de droite du Tableau II ci-dessous).

**TABLEAU II**

| | [prot] g/l | Activité | Activité spécifique (AS) | Rapport AS fraction/ AS lait Tg |
|---|---|---|---|---|
| Lait WT | 126 | 1193 | 9 | - |
| Lait Tg | 92,73 | 12946 | 140 | 1 |
| Fraction #2 | 2,47 | 3720 | 1506 | 11 |
| Fraction #4 | 14,31 | 6474 | 452 | 3 |
| Fraction #32 | 0,86 | 11 | 13 | 0 |
| Fraction #44 | 2,03 | 4522 | 2228 | 16 |
| Fraction #48 | 1,51 | 4220 | 2795 | 20 |
| Fraction #53 | 3,67 | 5411 | 1474 | 11 |
| Fraction #68 | 0,425 | 2586 | 6085 | 44 |

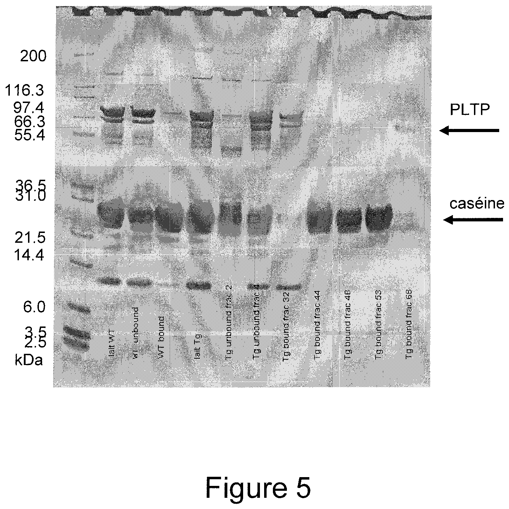

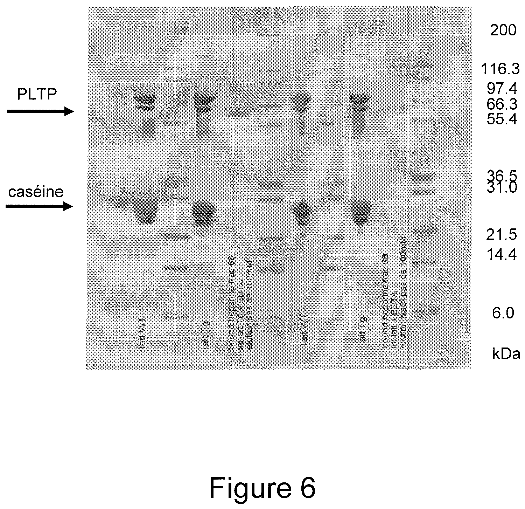

L'analyse électrophorétique en conditions dénaturantes montre à nouveau une bande principale à 60 kDa correspondant à la PLTP, et une bande plus discrète à 30 kDa correspondant aux caséines (Figure 5). Une seconde électrophorèse avec coloration moins intense des bandes montre une bande unique de 60 kDa pour la fraction #68 la plus active (Figure 6). Ces résultats indiquent que la PLTP humaine est le constituant majeur de la fraction présentant la plus forte activité spécifique de transfert des phospholipides.

### EXEMPLE 6 : UTILISATION DE LA PLTP HUMAINE OBTENUE À PARTIR DU LAIT DE LAPINES TRANSGÉNIQUES DANS LA PRÉVENTION DU CHOC ENDOTOXINIQUE CHEZ LA SOURIS PLTP-KNOCK-OUT.

Des données antérieures (GAUTIER et al., J Biol Chem, 283, 18702-10, 2008) ont montré que l'invalidation du gène endogène de la PLTP chez la souris (souris PLTP-KO homozygotes) conduit à une diminution de la capacité de neutralisation et de détoxification des LPS par rapport aux souris de type sauvage WT. La mortalité après injection d'une dose létale de LPS intervient plus précocement chez les souris PLTP-KO que chez les souris WT.

Afin de déterminer si l'apport exogène de PLTP recombinante peut améliorer la détoxification des LPS et la résistance au choc endotoxinique chez l'animal, des souris PLTP-KO ont reçu par voie intrapéritonéale une injection létale (15 mg/kg) ou sublétale (5 mg/kg) de LPS au temps 0, suivie d'injections intraveineuses successives de tampon PBS ou de PLTP recombinante (100 microlitres de fraction active #68) préparée à partir du lait de lapines transgéniques comme décrit à l'Exemple 5 ci-dessus. Les plasmas ont ensuite étés prélevés par ponction rétroorbitale pour doser les concentrations circulantes de cytokines (IL-6, IL-10, MCP-1, IFNgamma, TNFalpha), et de l'activité PLTP de transfert des phospholipides, selon les protocoles décrits par GAUTIER *et al.* (2008, précité). Enfin, les taux de mortalité ont été enregistrés sur une période de 4 jours suivant l'injection initiale de LPS.

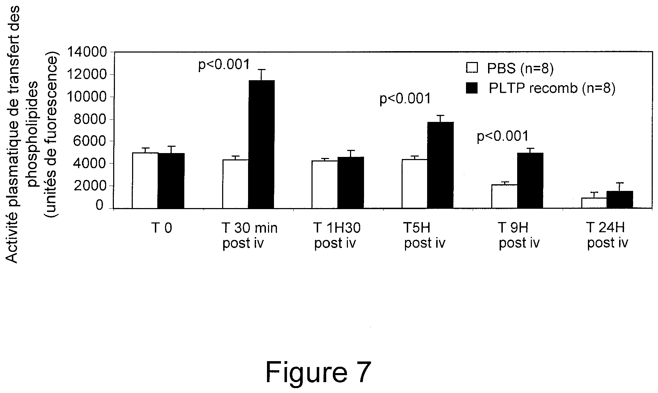

La Figure 7 illustre l'activité plasmatique de transfert des phospholipides chez des souris, après injection(s) de PLTP recombinante ou de tampon PBS. Cette activité plasmatique de transfert des phospholipides est significativement plus élevée 30 minutes après injection de PLTP recombinante par rapport aux souris recevant le tampon PBS. L'incrément d'activité disparait 90 minutes après la première injection de PLTP, mais reste significatif 60 minutes après la troisième injection qui est intervenue au temps 4 heures. De même, au temps t=9h, l'activité plasmatique de transfert des phospholipides est significativement plus élevée chez les animaux recevant la PLTP recombinante par rapport aux animaux recevant le tampon PBS (soit 60 minutes après la dernière injection de PLTP exogène au temps t=8h). Au temps t=24 heures, les animaux ayant reçu la dernière injection de PLTP recombinante au temps t=8h ne présentent plus d'incrément d'activité plasmatique de transfert des phospholipides par rapport aux animaux ayant reçu le tampon PBS.

Ces résultats indiquent que l'injection de PLTP exogène chez la souris PLTP-KO conduit à une élévation significative et transitoire de l'activité plasmatique de transfert des phospholipides avec une rémanence de l'ordre de 60 minutes.

Pour étudier les effets de la PLTP recombinante sur la production des cytokines IL-6, IL-10, MCP-1, IFNgamma, et TNFalpha, des animaux ayant reçu une injection intra-péritonéale unique de LPS à dose sub-létale (5 mg/kg), ont ensuite reçu plusieurs injections intraveineuses (veine caudale) de tampon PBS ou de PLTP recombinante, 10 min, 2h, 4h, 6h et 8h après l'administration des LPS. Des prélèvements sanguins rétroorbitaux ont été réalisés aux temps T0, T30min, T1h30, T5h, T9h et T24h chez les animaux anesthésiés à l'isoflurane, et les cytokines ont été dosées.

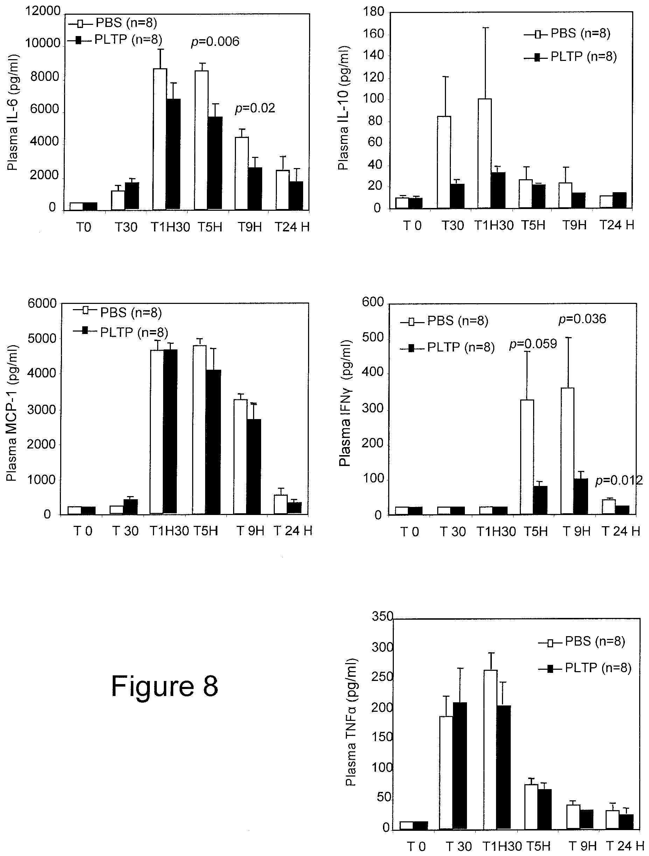

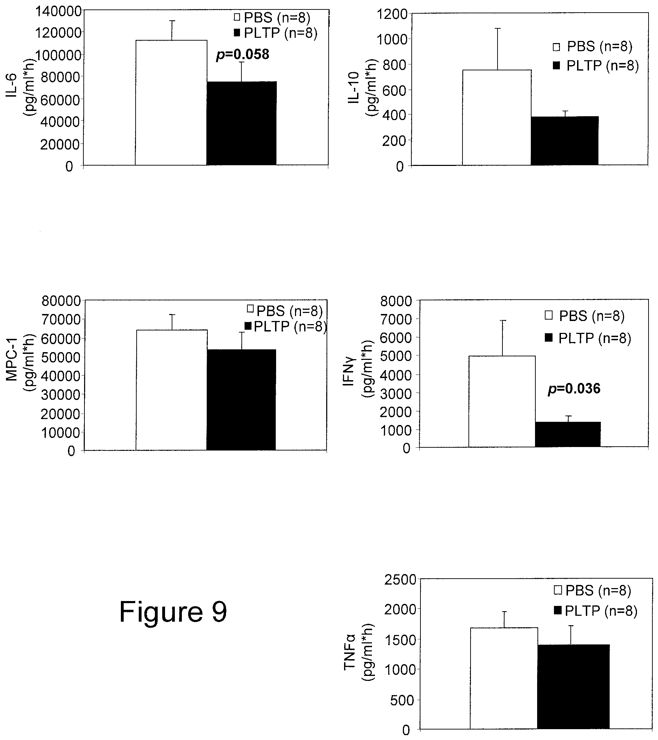

Les résultats sont illustrés par les Figures 8 et 9.

Après injection de la dose unique de LPS à 5 mg/kg, les concentrations circulantes de cytokines suivent une évolution biphasique au cours de la période de 24 heures étudiée, avec une phase initiale d'incrément, suivie d'une phase de décroissance conduisant au temps t=24h aux valeurs du bruit de fond mesurées chez les souris PLTP-KO au temps t=0 (Figure 8). Une diminution significative des concentrations circulantes d'IL-6, d'IL-10 et d'IFNgamma est observée à différents temps chez les souris recevant la PLTP recombinante par rapport aux souris recevant le PBS. Les cytokines IL-6, IL-10, MCP-1, IFNγ et TNF montrent des courbes biphasiques, avec production d'IL-6 et d'IFNγ significativement plus faible. Le calcul des aires sous la courbe (AUC) au cours de la période de 24 heures étudiée montre des valeurs d'IL-6 (p=0,058) et d'IFNgamma (p=0,036) significativement plus faibles chez les souris recevant la PLTP recombinante par voie intraveineuse que chez celles ayant reçu le PBS (Figure 9).

Afin d'étudier l'effet de la PLTP exogène sur la mortalité, l'injection intrapéritonéale d'une dose létale unique de LPS à 15 mg/kg a été suivie d'une séquence d'injections intraveineuses de PLTP exogène aux temps t=15 min, t=1h, t=2h, t=3h, t=4h, t=5h, t=7h après injection des LPS. Des prélèvements sanguins rétroorbitaux ont été réalisés chez les animaux anesthésiés aux temps T0, T1h30 et T6h après injection des LPS, et l'activité plasmatique de transfert des phospholipides a été déterminée. La durée de survie des animaux a été notée en parallèle.

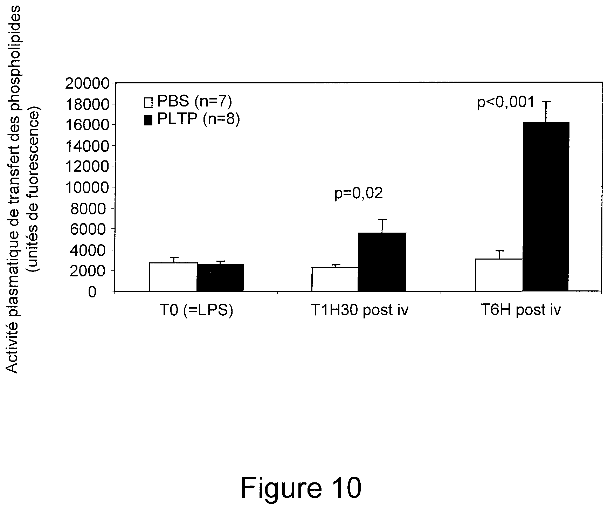

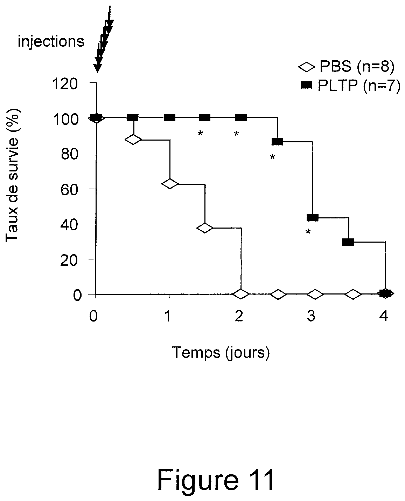

Les résultats sont illustrés par les Figures 10 et 11.

Comme le montre la Figure 10, l'activité plasmatique de transfert des phospholipides est significativement plus élevée 30 minutes après la seconde injection chez les souris recevant la PLTP recombinante par rapport aux souris recevant le tampon PBS. Un incrément d'activité (de l'ordre de 5 fois) est observé au temps t=6h, soit 1 heure après la 4^{ième} injection de PLTP recombinante. A nouveau, ces résultats indiquent que l'injection cumulée de PLTP exogène chez la souris PLTP-KO conduit à une élévation significative et transitoire de l'activité plasmatique de transfert des phospholipides avec une rémanence d'au moins 60 minutes. Les courbes de Kaplan-Meier (Figure 11) montrent le prolongement de la survie des animaux ayant reçu la PLTP recombinante exogène par rapport aux animaux ayant reçu le tampon PBS. Les durées moyennes de survie sont de 33,6±5,4 h (moyenne±sem) pour les souris ayant reçu le PBS (n=8) et de 77,1±6,2 h (moyenne±sem) pour les souris (n=7) ayant reçu la PLTP (p<0,05 versus PBS par le test de Chi2).

Ces résultats mettent en évidence l'effet bénéfique d'un apport en PLTP exogène chez l'animal soumis à un choc endotoxinique par injection intrapéritonéale unique de LPS. Des injections successives de PLTP au cours des premières heures suivant l'injection de LPS diminuent significativement la production de cytokines inflammatoires et allongent la survie.

### EXEMPLE 7 : UTILISATION DE LA PLTP HUMAINE, ACTIVE OU INACTIVEE, OBTENUE À PARTIR DU LAIT DE LAPINES TRANSGÉNIQUES DANS LA PRÉVENTION DU CHOC ENDOTOXINIQUE CHEZ LA SOURIS PLTP-KNOCK-OUT.

Afin de confirmer l'effet de la PLTP exogène sur la mortalité, observé à l'Exemple 6, l'injection intrapéritonéale d'une dose létale unique de LPS (25 mg/kg de poids corporel) a été suivie d'une séquence d'injections intraveineuses de PLTP recombinante (200 microlitres, 2,2 g protéines/l), préparée à partir du lait de lapines transgéniques comme décrit à l'Exemple 5, aux temps t=1h, 5h, 10h, 24h, 32h, 48h, 56h, 72h et 80h après l'injection de LPS.

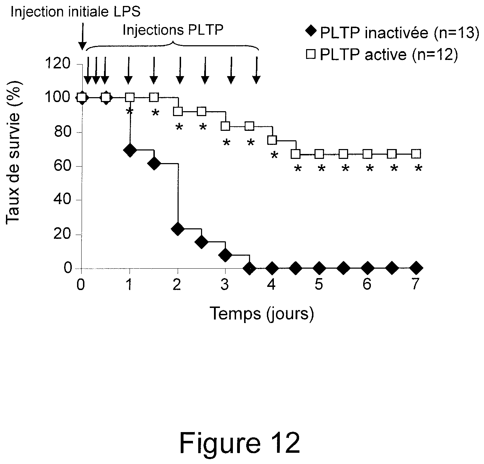

Le taux de survie a été suivi en fonction du temps, comme représenté à la Figure 12. L'effet de la PLTP recombinante active a été comparé à celui d'une PLTP inactivée par un chauffage d'une préparation de PLTP recombinante, purifiée selon l'Exemple 5, pendant 2 heures à 65°C. Les courbes Kaplan-Meier ont été comparées par le test de Chi2. Après 4 jours, la totalité des souris injectées avec le LPS puis la PLTP inactivée sont mortes. L'injection de PLTP recombinante active permet d'augmenter très significativement (p<0,05 versus PLTP inactivée) la survie des animaux. Plus de 60% des souris sont définitivement sauvées (au-delà de 7 jours). L'injection intraveineuse de PLTP recombinante exogène active, par opposition à la même PLTP inactivée par chauffage, augmente donc la survie des souris PLTP-KO ayant reçu une injection d'une dose létale de LPS.

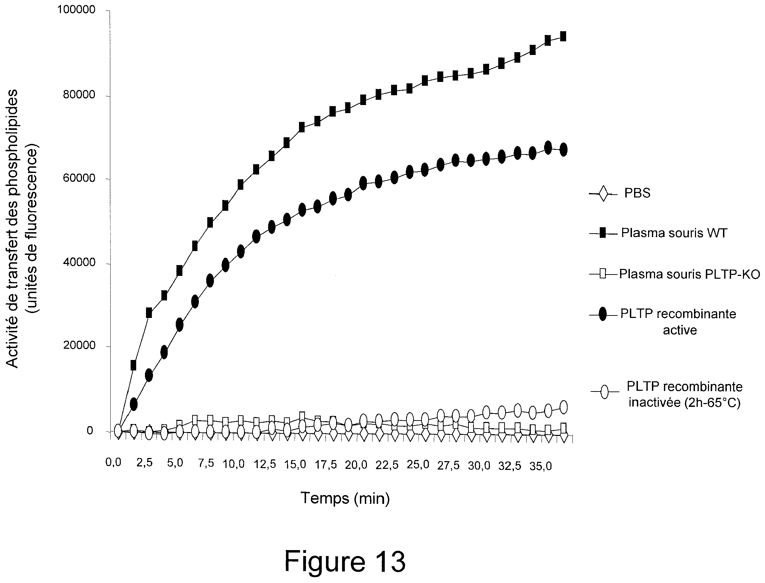

L'activité de transfert des phospholipides de la PLTP inactivée a été mesurée en fluorescence à l'aide d'un kit commercial (Roar Biomedical Inc.), comme dans l'Exemple 4, et comparée à celles d'un tampon PBS, de la PLTP recombinante active, d'un plasma de souris wild-type (WT), et d'un plasma de souris PLTP-KO. Les résultats, illustrés par la Figure 13, montrent que seuls le plasma de souris WT et la PLTP recombinante active permettent de détecter une activité de transfert des phospholipides, la PLTP recombinante inactivée, comme le tampon PBS et le plasma de souris PLTP-KO, ne résultant en aucune activité significative.

Ces résultats confirment l'effet bénéfique d'un apport en PLTP exogène chez l'animal soumis à un choc endotoxinique par injection intrapéritonéale unique de LPS. Des injections successives de PLTP au cours des premières heures suivant l'injection de LPS allongent significativement la survie.

## Revendications

1. Lapine transgénique, **caractérisée en ce qu'**elle contient dans son génome une ou plusieurs copies d'un transgène comprenant un polynucléotide codant pour la protéine plasmatique de transfert des phospholipides (PLTP) humaine, placé sous contrôle transcriptionnel d'un promoteur permettant son expression spécifique dans les cellules des glandes mammaires dudit animal, et **en ce qu'**elle sécrète dans son lait de la PLTP humaine active.

2. Lait d'une lapine transgénique selon la revendication 1.

3. Procédé d'obtention d'une préparation de PLTP humaine recombinante à partir du lait selon la revendication 2, **caractérisé en ce qu'**il comprend la clarification du lait, ladite clarification comprenant une étape de purification en présence d'EDTA et à pH acide, et la récupération du lactosérum.

4. Procédé selon la revendication 3, **caractérisé en ce qu'**il comprend en outre l'extraction de la PLTP à partir du lactosérum.

5. Procédé selon la revendication 4, **caractérisé en ce que** l'extraction de la PLTP à partir du lactosérum est effectuée par chromatographie d'affinité sur héparine.

## Patentansprüche

1. Transgenes Kaninchen, **dadurch gekennzeichnet, dass** es in seinem Genom eine Kopie oder mehrere Kopien eines Transgens enthält, welches ein Polynucleotid, das für das humane plasmatische Phospholipid-Transferprotein (PLTP) codiert, umfasst, das unter die transkriptionelle Kontrolle eines Promotors gestellt ist, der seine spezifische Expression in den Zellen der Brustdrüsen des Tiers erlaubt, und dadurch, dass es aktives humanes PLTP in seine Milch sekretiert.

2. Milch eines transgenen Kaninchens gemäß Anspruch 1.

3. Verfahren zur Herstellung eines Präparates von rekombinantem humanem PLTP ausgehend von der Milch gemäß Anspruch 2, **dadurch gekennzeichnet, dass** es die Klärung der Milch, wobei die Klärung eine Reinigungsstufe in Gegenwart von EDTA und bei saurem pH umfasst, und die Gewinnung des Milchserums umfasst.

4. Verfahren gemäß Anspruch 3, **dadurch gekennzeichnet, dass** es außerdem die Extraktion des PLTP aus dem Milchserum umfasst.

5. Verfahren gemäß Anspruch 4, **dadurch gekennzeichnet, dass** die Extraktion des PLTP aus dem Milchserum durch Affinitätschromatographie an Heparin durchgeführt wird.

## Claims

1. A female transgenic rabbit **characterized in that**, it contains, in its genome, one or more copies of a transgene comprising a polynucleotide encoding the human phospholipid transfer protein (PLTP), placed under the transcriptional control of a promoter allowing specific expression thereof in the mammary gland cells of said animal, and **in that** it secretes active human PLTP in its milk.

2. The milk of a female transgenic rabbit according to claim 1.

3. A method to obtain a preparation of recombinant human PLTP from the milk of claim 2, **characterized in that** it comprises clarification of the milk, said clarification comprising a purification step in the presence of EDTA and at acid pH, and recovery of the whey.

4. The method according to claim 3, **characterized in that** it further comprises extraction of the PLTP from the whey.

5. The method according to claim 4, **characterized in that** the extraction of PLTP from the whey is performed by heparin affinity chromatography.
